# EUROPÄISCHE PATENTSCHRIFT

(11) **EP 0 602 499 B1**
(45) Veröffentlichungstag und Bekanntmachung des Hinweises auf die Patenterteilung: **05.03.1997**
(21) Anmeldenummer: 93119651.3
(22) Anmeldetag: 06.12.1993
(51) Int. Cl.: C07C 45/00, C07C 29/20, C07C 49/403, C07C 35/08

(54) **Verfahren zur Herstellung eines Gemisches von Cyclohexanon und Cyclohexanol**
Process for the preparation of a mixture of cyclohexanone and cyclohexanol
Procédé pour la préparation d'un mélange de cyclohexanone et de cyclohexanol

(30) Priorität: 18.12.1992 DE 4242947
(43) Veröffentlichungstag der Anmeldung: 22.06.1994
(73) Patentinhaber: BAYER AG, 51368 Leverkusen (DE)
(72) Erfinder: Scharschmidt, Jürgen, Dr., D-47802 Krefeld (DE); Mendoza-Frohn, Christine, Dr., D-40699 Erkrath-Hochdahl (DE); Buysch, Hans-Josef, Dr., D-47809 Krefeld (DE); Klotzbücher, Rainer, Dr., D-47800 Krefeld (DE)

(56) Entgegenhaltungen:
- EP-A- 0 012 153
- GB-A- 1 063 357
- NL-A- 6 511 221
- US-A- 3 932 514
- Chem. and Ind, 1989, Seiten 830-833

## Beschreibung

Vorliegende Erfindung betrifft ein Verfahren zur Herstellung von Cyclohexanon und Cyclohexanol durch Hydrierung von Phenol in der Gasphase an Palladium auf Trägern, das dadurch gekennzeichnet ist, daß man durch spez. Behandlung der Katalysatoren bei der Aktivierung und Regenerierung gezielt bestimmte Verhältnisse von Cyclohexanon zu Cyclohexanol entsprechend dem Bedarf einstellen kann.

Bekanntlich wird Cyclohexanol hauptsächlich zur Herstellung von Adipinsäure und Cyclohexanon im wesentlichen für die Synthese von Caprolactam verwendet. Beide Ausgangsprodukte wiederum können durch Hydrierung von Phenol gewonnen werden. Wenn über diesen Weg die Versorgung einer Adipinsäure- und Caprolactamfabrikation erfolgen soll und beide wechselnde Auslastungen aufweisen, so ist man gezwungen, entweder Phenol ausschließlich zu Cyclohexanol zu hydrieren, was beispielsweise mit bestimmten Nickelkatalysatoren gelingt (vgl. Ullmann's Encyclopedia Vol. A 8, S. 218; DD 224315-A und DD 281079-A) und dann einen Teil des Cyclohexanols zu dehydrieren, beispielsweise mit Cu-, Zn- oder Cr-Katalysatoren (vgl. Ullmann's Encyclopedia Vol. A8, S. 221; Chem. and Ind. 1989, S. 832 und Chem. Techn. 18 (1966), S. 611 oder aber man hydriert einen Teil des Phenols an Nickelkatalysatoren zu Cyclohexanol und einen anderen Teil an Palladiumkatalysatoren zu Cyclohexanon. Eine solche Arbeitsweise wird u.a. in Chem. and Ind. 1989, S. 832, beschrieben.

In jedem Fall werden zwei verschiedene Katalysatoren und zwei dafür geeignete Anlagen benötigt.

Die Aufgabe bestand also darin, beide Ausgangsprodukte Cyclohexanon und Cyclohexanol an nur einem Katalysator und in nur einer Anlage in vorbestimmten, aber veränderlichen Mengenverhältnissen, entsprechend dem Bedarf, herzustellen.

Es wurde nun überraschend gefunden, daß diese Aufgabe auf einfache Weise gelöst werden kann, wenn man die an sich für eine selektive Hydrierung von Phenol zu Cyclohexanon üblichen Palladiumkatalysatoren nach besonderen Verfahrensweisen aktiviert und regeneriert.

Gegenstand der vorliegenden Erfindung ist demnach ein Verfahren zur Herstellung von Gemischen aus Cyclohexanon und Cyclohexanol mit vorbestimmten Verhältnissen zueinander im Bereich von 30:70 bis 98:2 durch Hydrierung von Phenol mit Wasserstoff in der Gasphase an Palladium/Träger-Katalysatoren, in dem man durch gezielte Durchführung der Erstaktivierung und der Regenerierung wahlweise ein Verhältnis von Cyclohexanon:Cyclohexanol = 85:15 bis 98:2 oder = 30:70 bis unterhalb 85:15 einstellen kann.

Die Erfindung betrifft demnach in einem ersten Aspekt ein Verfahren zur Herstellung von Gemischen aus Cyclohexanon und Cyclohexanol im Bereich von 85:15 bis 98:2 durch Hydrierung von Phenol mit Wasserstoff in der Gasphase bei 150 bis 250°C und 0,8 bis 8 bar, bevorzugt 1 bis 5 bar, an Palladium/Träger-Katalysatoren, das dadurch gekennzeichnet ist, daß man die Katalysatoren zur Erstaktivierung und zur Regenerierung nach Gebrauch gegebenenfalls einer Sauerstoff-Vorbehandlung und dann einer Wasserstoff-Behandlung bei jeweils 0,8 bis 8 bar, bevorzugt 1 bis 5 bar, besonders bevorzugt 1 bis 3 bar, unterwirft, wobei folgende Zeit-Temperaturbedingungen angewandt werden:
- zur Erstaktivierung keine O₂-Vorbehandlung und H₂-Behandlung während 50-10 h bei 300-500°C und
- zur Regenerierung O₂-Vorbehandlung während 30-0,5 h bei h 220-380°C und H₂-Behandlung während 2-6 h bei 150-250°C.

Die Erfindung betrifft weiterhin in einem zweiten Aspekt ein Verfahren zur Herstellung von Gemischen aus Cyclohexanon und Cyclohexanol im Bereich von 30:70 bis unterhalb 85:15 durch Hydrierung von Phenol mit Wasserstoff in der Gasphase bei 150 bis 250°C und 0,8 bis 8 bar, bevorzugt 1 bis 5 bar, an Palladium/Träger-Katalysatoren, das dadurch gekennzeichnet ist, daß man die Katalysatoren zur Erstaktivierung und zur Regenerierung nach Gebrauch einer Sauerstoff-Vorbehandlung und dann einer Wasserstoff-Behandlung bei jeweils 0,8 bis 8 bar, bevorzugt 1 bis 5 bar, besonders bevorzugt 1 bis 3 bar, unterwirft, wobei folgende Zeit-Temperatur-Bedingungen angewandt werden:
- zur Erstaktivierung O₂-Vorbehandlung während 40-3 h bei 250-500°C und H₂-Behandlung während 2-6 h bei 150-250°C und
- zur Regenerierung O₂-Vorbehandlung während 30-0,5 h bei 380-600°C und H₂-Behandlung während 2-6 h bei 150-250°C.

Die Verhältnisse Cyclohexanon (Mol-Gew. 98) zu Cyclohexanol (Mol-Gew.100) sind auf die Massen bezogen, gelten aber wegen der eng beieinander liegenden Molgewichte und anderer physikalischer Daten nahezu identisch auch für Mol- oder Volumenverhältnisse.

Diese gezielte und flexible Einstellung der Phenolhydrierung auf verschiedene Cyclohexanon/Cyclohexanol-Gemische war nicht zu erwarten. Bei der Hydrierung an Nickelkatalysatoren erhält man in der Regel ganz überwiegend Cyclohexanol und an Palladiumkatalysatoren Cyclohexanon (Ullmann's Encyclopedian loc.cit.)

An Palladiumkatalysatoren wird normalerweise zu Beginn der Phenolhydrierung eine Übergangsphase beobachtet, in der zuerst nennenswerte Mengen an Cyclohexanol neben dem Hauptprodukt Cyclohexanon entstehen, die im Laufe der Zeit zurückgehen, bis der Katalysator "eingefahren" ist (vgl. z.B. DD-PS 242039, S. 1).

Man hat diese Erscheinung mit anfänglich vorliegenden, langsam desaktivierenden "überaktiven" Zentren in Zusammenhang gebracht, die durch ein als Moderator eingesetztes Katalysatorgift, wie CO, gezielt desaktiviert werden sollen (vgl. DD-PS 150999).

Erkenntnisse und Verfahrensweisen zu einer gezielten Modifizierung von Palladiumkatalysatoren hinsichtlich der hier gestellten Aufgabe sind bisher nicht bekannt geworden.

Dies gelingt jedoch in nicht vorhersehbarer Weise durch das erfindungsgemäße Verfahren.

In der Publikation Chem. and Ind. 1989, S. 831, wird sogar angegeben und experimentell belegt, daß Regenerierungen bei höheren Temperaturen, wie sie erfindungsgemäß vorgeschlagen werden, zu wenig effektiv seien und zu einer raschen Desaktivierung führen; eine ähnliche Angabe macht Chem. Techn. 29 (1977), S. 41, nach der eine optimale Aktivierung und Regenerierung bei 170-185 C erfolgen soll.

Geeignete Ausgangsprodukte für das erfindungsgemäße Verfahren sind Phenol und Wasserstoff. Phenol muß eine besondere Reinheit aufweisen, wie sie üblicherweise bei Phenolqualitäten, die nach dem sogenannten Hock-Verfahren gewonnen werden, gegeben ist. Besonders Katalysatorgifte, wie Stickstoff-, Schwefel- und Quecksilberverbindungen und auch CO und CO-Generatoren, wie Ameisen- und Oxalsäure, sollen im Phenol nicht oder nur in geringen Mengen unter 10 ppm, besser unter 1 ppm vorhanden und noch besser nicht mehr nachweisbar sein, wenn in den herzustellenden Gemischen größere Mengen an Cyclohexanol erwartet werden. Will man jedoch eine besonders hohe Selektivität für Cyclohexanon erzielen, können Spuren an Schwefelverbindungen oder CO bzw. CO-Generatoren, vorzugsweise CO oder CO-Generatoren im Bereich von 2-150, vorzugsweise 10-100 ppm zugesetzt werden (vgl. DD-PS 150999, US 4 520 129).

Ähnliche Einschränkungen wie für Phenol gelten für Wasserstoff. Die oben angegebenen Katalysatorgifte dürfen nur in den genannten Grenzen vorhanden, besser jedoch nicht mehr nachweisbar sein. Allerdings können Inertgase wie Kohlenwasserstoffe, Stickstoff Edelgase oder Kohlendioxid durchaus akzeptiert werden. Aus ökonomischen Gründen kann es jedoch sinnvoll sein, auf solche Beimengungen zu verzichten, da sie regelmäßig aus dem Kreisgasstrom entfernt werden müssen und mit ihnen jeweils große Mengen an Wasserstoff die dann gegebenenfalls abgetrennt werden müssen. Wasserstoff wird in einer Menge von 3-30 Mol/Mol Phenol, bevorzugt 3,5-10 Mol/Mol eingesetzt.

Phenol und Wasserstoff können auch Cyclohexanol und/oder Cyclohexanon beige-mischt werden, je nachdem welches der Produkte im Überschuß entstanden ist und aktuell nicht verwertet, durch Rückführung aber in das jeweils verlangte Produkt umgewandelt werden kann.

Palladiumkatalysatoren, die für das erfindungsgemäße Verfahren geeignet sind, sind diejenigen, die üblicherweise für eine Hydrierung von Phenol zu Cyclohexanon verwendet werden, also Katalysatoren, die Palladium auf bekannten Trägern abgeschieden enthalten.

Geeignet sind beispielsweise solche Katalysatoren wie sie in DE-OS 1 298 098, DE-OS 2 059 938, DE-OS 2 025 726, DE-OS 1 290 538, DD 150 999, DD 92 243, EP 12 153, DE-OS 2 606 489, DE-AS 2 045 882, DE-OS 1 952 208, DE-OS 2 025 726, Chem. and Ind. 1989, S. 830-2, Chem. Techn. 29 (1977), S. 38-41 beschrieben sind. Besonders geeignet sind solche Katalysatoren, die Trägermaterialien aus der Gruppe der Aluminiumoxide, bevorzugt γ-Al₂O₃, Böhmit oder α-Al₂O₃, Siliciumdioxide, Siliciumdioxid-Aluminiumoxid-Gemische, besonders Alumosilikate, amorphe Kieselsäuren, Kieselgure, Barium-, Strontium-, Calcium-und Magnesiumcarbonate, Mischungen dieser Verbindungen, gegebenenfalls unter Zusatz von Silicium- oder Aluminiumoxiden, Magnesiumoxide, Titanoxide, Zirkonoxide, Magnesiumsilikate, Zirkonsilicate, Spinelle aus Alkali- und Erdalkalialuminaten, bevorzugt solche auf der Basis von Lithium und Barium enthalten. Bevorzugt sind solche Katalysatoren, die unter Verwendung von Alkali- und Erdalkalibasen hergestellt wurden, und in denen Palladium in der äußeren Schicht des Trägers schalenförmig konzentriert vorliegt.

Das entsprechend den genannten Literaturstellen auf Träger aufgebrachte Palladium kann zusätzlich andere Edelmetalle, wie Platin, Rhodium, Iridium oder Ruthenium, in Mengen von bis zu 20 Gew.-%, bevorzugt bis zu 10 Gew.-% enthalten; besonders bevorzugt wird jedoch Palladium allein verwendet.

Die Katalysatoren können in den verschiedensten Formen zur Anwendung kommen, z.B. als Kugeln, Granulate, Extrudate, Tabletten, Sattelkörper, Raschigringe, Bruchstücke oder Wabenkeramik.

Vor Durchführung der Phenolhydrierung muß der Katalysator aktiviert werden. Dies geschieht, wenn hohe Selektivitäten für Cyclohexanon, also Gemische von Cyclohexanon zu Cyclohexanol von 85:15 bis 98:2 gewünscht werden, dadurch, daß man den Katalysator 50-10 h bei 300-500°C, bevorzugt 40-20 h bei 350-450°C, besonders bevorzugt 35-20 h bei 370-450°C mit Wasserstoff enthaltenden Gasen, besonders Wasserstoff-Stickstoff-Gemischen oder mit reinem Wasserstoff behandelt. Das Ergebnis dieser Aktivierung ist besonders überraschend, da die ansonsten übliche Einfahrzeit erfindungsgemäß erheblich verkürzt ist gegenüber der Einfahrzeit bei der in der Literatur empfohlenen Aktivierung bei ca. 180°C.

Nach einer gebrauchsbedingten Desaktivierung erfolgt eine Regenerierung des Katalysators durch Überleiten von Inertgas(z.B. Stickstoff)-Sauerstoff-Gemischen je nach Ausmaß der Verkokung während 30-0,5 h bei 220-380°C, vorzugsweise während 25-1 h bei 230-370°C, bes. bevorzugt während 20-1 h bei 250-360°C, wobei der Sauerstoffgehalt im Gemisch von gegebenenfalls weniger als 1 Vol.%, z.B. 0,5 Vol.-%, entsprechend der Menge an abzubrennenden organischen Ablagerungen und den Möglichkeiten zur Abführung der Verbrennungswärme langsam erhöht wird auf bis zu 30 Vol.%, bevorzugt auf den Gehalt von 21 Vol.-% der atmosphärischen Luft. Demzufolge kann hierzu atmosphärische Luft zunächst mit Inertgas (z.B. N₂) auf 0,5 Vol.-% O₂ verdünnt werden und diese Verdünnung durch Zurücknahme des Inertgasanteils reduziert werden, bis der Normalgehalt von etwa 21 Vol.-% erreicht ist. Zur Erreichung von bis zu 30 Vol.-% O₂ kann atmosphärische Luft mit O₂ angereichert werden.

Werden dagegen Gemische mit höheren Anteilen an Cyclohexanol gewünscht, also von mehr als 15 % bis 70 %, so wird die Aktivierung des Palladiumkatalysators zuerst durch Überleiten von Sauerstoff-Stickstoff-Gemischen mit Gehalten an Sauerstoff bis zu 21 Vol.%, gegebenenfalls auch höheren, während 40-3 h bei 250-500°C, vorzugsweise während 30-4 h bei 300-450°C, besonders bevorzugt während 24-5 h bei 350-425°C und dann durch Überleiten von Wasserstoff enthaltenden Gasen oder reinem Wasserstoff bei 150-250°C während 2-6 h vorgenommen.

Die Regenerierung eines desaktivierten Katalysators, der hohe Cyclohexanolanteile produzieren soll, erfolgt durch Überleiten von Sauerstoff-Stickstoff-Gemischen je nach den Gegebenheiten, ob viel oder wenig organische Substanz abgelagert wurde, mit Sauerstoffgehalten unter 1 Vol.% bis zu solchen von 21 Vol.% oder auch höheren, denen man sich im Regenerierprozeß langsam annähern kann, während 30-0,5 h bei 380-600°C, vorzugsweise während 25-1 h bei 390-580°C, besonders bevorzugt während 20-1 h bei 400-550°C und anschließend durch Überleiten von Wasserstoff enthaltenden Gasen oder reinem Wasserstoff bei Temperaturen von 150 bis 250°C in 2-6 h.

Die O₂-Vorbehandlung und die H₂-Behandlung werden bei einem Druck von 0,8 bis 8 bar, bevorzugt 1 bis 5 bar, besonders bevorzugt bei 1 bis 3 bar, vorgenommen.

Diese Druckangaben beziehen sich jeweils auf den Gesamtdruck der O₂-Inertgas- bzw. H₂-Inertgasgemische.

Die erfindungsgemäße Erstaktivierung und die gebrauchsbedingten Regenerierungen können selbstverständlich in einem separaten Reaktionsgefäß durchgeführt werden. Den vollen wirtschaftlichen Nutzen erreicht man jedoch dann, wenn diese Behandlungsvorgänge im Hydrierreaktor zur Herstellung des Cyclohexanon/Cyclohexanol-Gemisches durchgeführt werden. Solche Reaktoren sind die dem Fachmann bekannten, wie Festbettreaktoren, in denen der Katalysator in Röhren oder auf Böden, gegebenenfalls im Gemenge mit inerten Füllkörpern angebracht ist, oder Fließbettreaktoren.

Vor Beginn der H₂-Behandlung wird der Sauerstoff der vorangegangenen O₂-Vorbehandlung durch nichtbrennbare Inertgase, wie sie zur Abmischung des O₂ eingesetzt werden (z.B. N₂, CO₂, Edelgase), völlig verdrängt.

Die im Rahmen einer Regenerierung anschließende H₂-Behandlung kann als separater Schritt ausgeführt werden. In bevorzugter Weise wird die H₂-Behandlung unter Hydrierbetriebsbedingungen vorgenommen also simultan zur Wiederaufnahme der Phenolhydrierung.

Die Phenolhydrierung wird bei 150 bis 250°C und 0,8 bis 8 bar, bevorzugt 1 bis 5 bar, durchgeführt.

### Beispiele

### Beispiel 1a (nicht erfindungsgemäß)

### Herstellung des Katalysators

Tranken von Li-Al-Spinell mit Na₂PdCl₄ (18 g/l), Reduzieren mit Hydrazin, Waschen, Trocknen (gemäß Beispiel 1 a aus DE-AS 2.045.882).

### Beispiel 1b (nicht erfindungsgemäß)

Der nach Beispiel 1a erhaltene Katalysator wurde in einer technischen Anlage zur Herstellung von Cyclohexanon eingesetzt, wobei Phenolumsätze >99,9 % erzielt wurden. Nach ca. 6 000 h Laufzeit stieg der Phenolgehalt im Rohprodukt auf über 1 000 ppm an. Die Produktion wurde unterbrochen zur Entnahme von Proben des desaktivierten Katalysators. Er lieferte während der zweiten Periode seiner Laufzeit ein Cyclohexanor/Cyclohexanol-Verhältnis von 19:1 (siehe Beispiel 1 b der DE-AS 2 045 882).

### Beispiel 1c (erfindungsgemäß)

Der Katalysator aus Beispiel 1a wurde 24 h bei 400°C in einem Gemisch aus 10 % H₂ und 90 % N₂ (vol-%) reduziert und dann mit 1,0 g Phenol/ml Kat x h und 4 Mol Wasserstoff/Mol Phenol bei 150°C belastet. Bei vollständigem Phenolumsatz und einer Selektivität von 99,8 % erhielt man Cyclohexanon und Cyclohexanol im Verhältnis von 12,0 : 1.

Nach 600 h Laufzeit bei praktisch gleichbleibendem Cyclohexanon/Cyclohexanol-Verhältnis wurde der Versuch abgebrochen.

Dieser Versuch belegt, daß durch eine besondere Vorbehandlung des frischen Katalysators mit wasserstoffhaltigen Gasen im Gegensatz zum Stand der Technik (siehe DE-AS 2 045 882, Beispiele 1 a und 1 b) ein bestimmtes anderes Verhältnis von Cyclohexanon zu Cyclohexanol eingestellt werden kann.

### Beispiel 2 (erfindungsgemäß)

Der Katalysator aus Beispiel 1a wurde 16 h bei 350°C im Luftstrom erhitzt, nach Verdrängen von Luft bei 150°C mit Stickstoff-Wasserstoff (Vol. 90: 10) während 4 h reduziert und dann mit 1,0 g Phenol/ml Kat. x h und 4 Mol Wasserstoff/Mol Phenol bei 150°C belastet. Bei vollständigem Phenolumsatz und einer Selektivität von 99,9 % erhielt man Cyclohexanon und Cyclohexanol im Verhältnis von 1,5. Auch bei geringerer Belastung von etwa 0,7 g Phenol/mi Kat. x h sank der Wert nur unwesentlich von 1,5 auf 1,3. Nach 600 h Laufzeit bei praktisch gleichbleibendem Cyclohexanon/Cyclohexanol-Verhältnis wurde der Versuch abgebrochen.

Dieser Versuch belegt, daß eine besondere Vorbehandlung des frischen Katalysators mit sauerstoffhaltigen Gasen ein bestimmtes Verhältnis von Cyclohexanon zu Cyclohexanol eingestellt werden kann. Der Vergleich mit Beispiel 1 lehrt, daß aus demselben Kontakt je nach Vorbehandlung eine Einstellung auf verschiedene Produktzusammensetzungen möglich ist.

### Beispiel 3 a (nicht erfindungsgemäß)

Ein α-Al₂O₃-Träger (SPH 512, Rhône-Poulenc) wurde mit NaOH getränkt, getrocknet und dann mit Na₂PdCl₄ getränkt, mit Hydrazin reduziert, gewaschen und getrocknet. Die Belegung mit Pd betrug 18 g/l Kat. (gemäß Beispiel 1 a aus DE-AS 2 045 882).

### Beispiel 3 b (erfindungsgemäß)

Der Katalysator aus Beispiel 3 a wurde 24 h bei 400°C in einem Gemisch aus 10% H₂ und 90 % N₂ (vol-%) reduziert und dann mit 1,0 g Phenol/ml Kat. x h und 4 Mol Wasserstoff/Mol Phenol bei 150°C belastet. Bei vollständigem Phenolumsatz und einer Selektivität von 99,9 % erhielt man schon nach 100 h Laufzeit Cyclohexanon und Cyclohexanol im Verhältnis von 47 : 1.

Nach 600 h Laufzeit bei praktisch gleichbleibendem Cyclohexanon/Cyclohexanol-Verhältnis wurde der Versuch abgebrochen.

Dieser Versuch belegt, daß durch eine besondere Vorbehandlung des frischen Katalysators mit wasserstoffhaltigen Gasen ein bestimmtes Verhältnis von Cyclohexanon zu Cyclohexanol eingestellt werden kann (vergleiche Beispiel 1 c und DE-AS 2 045 882, Beispiel 1 a und 1b).

### Beispiel 4

Der Katalysator aus Beispiel 3 a wurde 24 h bei 400°C in Luft behandelt und dann mit 1,0 g Phenol/ml Kat. x h und 4 Mol Wasserstoff/Mol Phenol bei 150°C belastet. Bei vollständigem Phenolumsatz und einer Selektivität von 99,8 % erhielt man Cyclohexanon und Cyclohexanol im Verhältnis von 3 : 1.

Nach 600 h Laufzeit bei praktisch gleichbleibendem Cyclohexanon/Cyclohexanol-Verhältnis wurde der Versuch abgebrochen.

Dieser Versuch belegt, daß durch eine besondere Vorbehandlung des frischen Katalysators mit sauerstoffhaltigen Gasen ein bestimmtes Verhältnis von Cyclohexanon zu Cyclohexanol eingestellt werden kann.

Der Vergleich mit Beispiel 3 lehrt, daß aus demselben Kontakt je nach Vorbehandlung eine Einstellung auf verschiedene Produktzusammensetzungen möglich ist.

### Beispiel 5 (erfindungsgemäß)

Der Katalysator aus Beispiel 1b wurde nach 6 000 h Laufzeit regeneriert durch Überleiten von Luft bei 350°C während 4 h und anschließend nach Verdrängen von Luft mit Stickstoff/Wasserstoff (Vol. 90 : 10) bei 150°C während 4 h reduziert. Bei einer Belastung von 1,0 g Phenol/ml Kat. x h, einer Temperatur von 150°C, einem Molverhältnis von Wasserstoff zu Phenol von 4:1 lieferte der regenerierte Kontakt einen Umsatz von etwa 97 %, eine Selektivität von 99,9 bis 99,98 % und nach einer Einfahrzeit von etwa 200 h ein Reaktionsprodukt mit einem Cyclohexanon/Cyclohexanol-Verhältnis von 16 bis 18. Nach einer Laufzeit von 2 800 h wurde bei praktisch unveränderten Ergebnissen der Versuch beendet.

Dieser Versuch zeigt, daß durch die erfindungsgemäße Regenerierung schon nach kurzer Einfahrzeit das gewünschte hohe Produktverhältnis von Cyclohexanon zu Cyclohexanol und außerdem eine lange Laufzeit bei sehr geringer Nebenproduktbildung erzielt werden.

### Beispiel 6 (erfindungsgemäß)

Der Katalysator aus Beispiel 1b wurde nach 6 000 h Laufzeit regeneriert durch Überleiten von Luft bei 500°C während 4 h und dann nach Verdrängen der Luft durch Stickstoff bei 150°C während 4 h mit einem Gemisch aus 10 % H₂/90 % N₂ reduziert.

Bei einer Belastung von 0,9 g Phenol/ml Kat. x h, einer Reaktionstemperatur von 150°C, einem Molverhältnis Wasserstoff zu Phenol von 4,5:1 erhielt man ohne wesentliche Änderung während 600 h Laufzeit vollständigen Umsatz, eine Selektivität von 99,7 bis 99,8% und ein Verhältnis von Cyclohexanon zu Cyclohexanol von 2,2 bis 2,4.

Der Vergleich mit Beispiel 5 lehrt, daß aus demselben desaktivierten Kontakt je nach Regenerierbedingungen eine Einstellung auf verschiedene Produktzusammensetzungen möglich ist.

### Beispiel 7a (nicht erfindungsgemäß)

Ein keramischer Träger mit 98 % α-Al₂O₃ und 2 % MgO (Hoechst-Ceram Tec, A 980) wurde mit einem Ba-Salz getränkt, getrocknet und kalziniert, so daß ein Ba-Spinell mit 6,5 % Ba entstand.

Dieser Ba-Spinell wurde gemäß Beispiel 1 a aus DE-AS 2 045 882 mit Natronlauge getränkt, getrocknet, mit Na₂PdCl₄ getränkt, mit Hydrazin reduziert, gewaschen und getrocknet. Der fertige Katalysator hat einen Gehalt von 8 g/l Pd.

### Beispiel 7b (nicht erfindungsgemäß)

Der nach 7 a erhaltene Kalatysator wurde in einer Technikumsanlage (1 l-Röhrenreaktor mit 4 cm Durchmesser) zur Herstellung von Cyclohexanon eingesetzt, wobei Phenolumsätze >99,9 % erzielt wurden. Nach 2 170 h Laufzeit stieg der Phenolgehalt im Rohprodukt auf über 1 000 ppm an. Der Versuch wurde zur Entnahme von Proben des desaktivierten Katalysators unterbrochen.

### Beispiel 7c (erfindungsgemäß)

Der Katalysator aus Beispiel 7b wurde regeneriert durch vierstündiges Überleiten von Luft bei 350°C und nach Verdrängen der Luft durch Stickstoff reduziert durch 4stündiges Überleiten von 10 % Wasserstoff/ 90 % N₂ bei 150°C. Im Molverhältnis von 4:1 wurden dann Wasserstoff und Phenol bei 150°C und einer Belastung von 1,0 g Phenol/ml Kat. x h über den Katalysator geleitet. Man erhielt nach einer Einfahrzeit von 150 h bei einem Umsatz von 95 bis 97% und einer Selektivität von 99,7 bis 99,9 % ein Cyclohexanon/Cyclohexanol-Verhältnis von 18 bis 30. Nach 2 600 h wurde der Versuch abgebrochen (vgl. Bemerkung zu Beispiel 5).

### Beispiel 8 a (nicht erfindungsgemäß)

Der nach 3 a erhaltene Katalysator wurde in einer Technikumsanlage (1 l-Röhrenreaktor mit 4 cm Durchmesser) zur Herstellung von Cyclohexanon eingesetzt, wobei Phenolumsätze >99,9 % erzielt wurden. Nach 900 h Laufzeit stieg der Phenolgehalt im Rohprodukt auf über 1 000 ppm an. Der Versuch wurde zur Entnahme von Proben des desaktivierten Katalysators unterbrochen.

### Beispiel 8 b (erfindungsgemäß)

Der Katalysator aus Beispiel 8 a wurde regeneriert durch 16 h Überleiten von Luft bei 500°C und nach Verdrängen der Luft durch Stickstoff reduziert durch 4-stündiges Überleiten eines Gemisches aus 10 vol-% H₂ und 90 % N₂ bei 150°C.

Im Molverhältnis von 4 : 1 wurden dann Wasserstoff und Phenol bei 150°C und einer Belastung von 1,0 g Phenol/ml Kat x h über den Katalysator geleitet. Man erhielt nach einer Einfahrzeit von 150 h bei einem Umsatz von 99,9% und einer Selektivität von 99,9% ein Cyclohexanon/Cyclohexanol-Verhältnis von 4 : 1. Nach 600 h wurde der Versuch abgebrochen (vergleiche Bemerkung zu Beispiel 6).

### Beispiel 9 (erfindungsgemäß)

Der Katalysator aus Beispiel 1 b wurde nach 6 000 h durch Überleiten von Luft bei 270°C in 4 h regeneriert und nach Verdrängen von Luft durch Stickstoff mit 10% Wasserstoff/ 90 % N₂ bei 150°C in 4 h reduziert. Im Molverhältnis 4:1 wurden Wasserstoff und Phenol bei einer Belastung von 1 g Phenol/ml Kat. x h bei 150°C übergeleitet. Infolge der relativ niedrigen Regenerierungstemperatur und relativ kurzen Regenerierungszeit verlängerte sich die Einfahrzeit des Katalysators auf 500 h, wobei ein Cyclohexanon/Cyclohexanol-Verhältnis von 1,3 bis 8 vorlag. Dann erreichte es bei 98 bis 99 % Umsatz und 99,7 bis 99,8 % Selektivität ein Cyclohexanon/Cyclohexanol-Verhältnis von 19 bis 21. Nach 2 600 h wurde der Versuch bei praktisch unveränderten Werten abgebrochen.

### Beispiel 10 (Vergleichsbeispiel)

Der Katalysator aus Beispiel 1 b wurde regeneriert durch 6 h Überleiten von Luft bei 185°C und nach Verdrängen der Luft durch Stickstoff reduziert durch 4-stündiges Überleiten eines Gemisches aus 10 vol-% H₂ und 90 % N₂ bei 150°C.

Im Molverhältnis von 4 : 1 wurden dann Wasserstoff und Phenol bei 150°C und einer Belastung von 1,0 g Phenol/ml Kat x h über den Katalysator geleitet. Man erhielt einen Umsatz von nur 97 % und bei einer Selektivität von 99,9 % ein Cyclohexanon/Cyclohexanol-Verhältnis von 4 : 1. Nach 600 h wurde der Verusch abgebrochen.

Dieser Versuch lehrt, daß 185°C entsprechend dem Stand der Technik (Chem. Techn. 29 (1977) S. 41) als Regenerierungstemperatur nicht geeignet ist, den Katalysator in seinen alten Zustand zurückzuversetzen (siehe Beispiel 1b). Dort wurde ein Umsatz von 99,9% und ein Cyclohexanon/Cyclohexanol-Verhältnis von 19 : 1 erzielt.

## Patentansprüche

1. Verfahren zur Herstellung von Gemischen aus Cyclohexanon und Cyclohexanol im Bereich von 85:15 bis 98:2 durch Hydrierung von Phenol mit Wasserstoff in der Gasphase bei 150 bis 250°C und 0,8 bis 8 bar, bevorzugt 1 bis 5 bar, an Palladium/Träger-Katalysatoren, dadurch gekennzeichnet, daß man die Katalysatoren zur Erstaktivierung und zur Regenerierung nach Gebrauch gegebenenfalls einer Sauerstoff-Vorbehandlung und dann einer Wasserstoff-Behandlung bei jeweils 0,8 bis 8 bar, bevorzugt 1 bis 5 bar, besonders bevorzugt 1 bis 3 bar, unterwirft, wobei folgende Zeit-Temperaturbedingungen angewandt werden:
- zur Erstaktivierung keine O₂-Vorbehandlung und H₂-Behandlung während 50-10 h bei 300-500°C und
- zur Regenerierung O₂-Vorbehandlung während 30-0,5 h bei 220-380°C und H₂-Behandlung während 2-6 h bei 150-250°C.

2. Verfahren zur Herstellung von Gemischen aus Cyclohexanon und Cyclohexanol im Bereich von 30:70 bis unterhalb 85:15 durch Hydrierung von Phenol mit Wasserstoff in der Gasphase bei 150 bis 250 C und 0,8 bis 8 bar, bevorzugt 1 bis 5 bar, an Palladium/Träger-Katalysatoren, dadurch gekennzeichnet, daß man die Katalysatoren zur Erstaktivierung und zur Regenerierung nach Gebrauch einer Sauerstoff-Vorbehandlung und dann einer Wasserstoff-Behandlung bei jeweils 0,8 bis 8 bar, bevorzugt 1 bis 5 bar, besonders bevorzugt 1 bis 3 bar, unterwirft, wobei folgende Zeit-Temperaturbedingungen angewandt werden:
- zur Erstaktivierung O₂-Vorbehandlung während 40-3 h bei 250-500°C und H₂-Behandlung während 2-6 h bei 150-250°C und
- zur Regenerierung O₂-Vorbehandlung 30-0,5 h bei 380-600°C und H₂-Behandlung während 2-6 h bei 150-250°C.

3. Verfahren nach Anspruch 1 und nach Anspruch 2, dadurch gekennzeichnet, daß zur O₂-Behandlung ein Inertgas-O₂-Gemisch eingesetzt wird, dessen O₂-Gehalt anfangs 0,5 Vol-% beträgt und während der O₂-Behandlung auf bis zu 30 Vol-% steigt.

4. Verfahren nach Anspruch 3, dadurch gekennzeichnet, daß der O₂-Anteil auf bis zu 21 Vol-% steigt.

5. Verfahren nach Anspruch 4, dadurch gekennzeichnet, daß atmosphärische Luft zunächst mit Inertgas verdünnt wird und während der O₂-Behandlung der Inertgasanteil reduziert wird, bis der Normalwert von 21 Vol-% O₂ erreicht ist.

6. Verfahren nach Anspruch 1 und nach Anspruch 2, dadurch gekennzeichnet, daß die O₂-Vorbehandlung und die H₂-Behandlung im Phenolhydrierungsreaktor durchgeführt werden.

7. Verfahren nach Anspruch 6, dadurch gekennzeichnet, daß die H₂-Behandlung im Rahmen einer Regenerierung unter den Hydrierbetriebsbedingungen vorgenommen wird.

8. Verfahren nach Anspruch 1, dadurch gekennzeichnet, daß die Erstaktivierung durch H₂-Behandlung während 40 bis 20 h bei 350 bis 450°C, bevorzugt während 35 bis 20 h bei 370 bis 450°C vorgenommen wird und die Regenerierung durch O₂-Behandlung während 25 bis 1 h bei 230 bis 370°C, bevorzugt während 20 bis 1 h bei 250 bis 360°C und anschließender H₂-Behandlung vorgenommen wird.

9. Verfahren nach Anspruch 2, dadurch gekennzeichnet, daß die Erstaktivierung durch O₂-Behandlung während 30 bis 4 h bei 300 bis 450°C, bevorzugt während 24 bis 5 h bei 350 bis 425°C und anschließender H₂-Behandlung vorgenommen wird und die Regenerierung durch O₂-Behandlung während 25 bis 1 h bei 390 bis 580°C, bevorzugt während 20 bis 1 h bei 400 bis 550°C und anschließender H₂-Behandlung vorgenommen wird.

## Claims

1. Process for the preparation of mixtures of cyclohexanone and cyclohexanol in the range from 85:15 to 98:2 by hydrogenation of phenol with hydrogen in the gas phase at 150 to 250°C under 0.8 to 8 bar, preferably 1 to 5 bar, over supported palladium catalysts, characterized in that, for initial activation and for regeneration after use, the catalysts are subjected to an oxygen pretreatment, if appropriate, and then a hydrogen treatment in each case under 0.8 to 8 bar, preferably 1 to 5 bar, particularly preferably 1 to 3 bar, the following time/temperature conditions being applied:
- for initial activation, no O₂ pretreatment and H₂ treatment for 50-10 hours at 300-500°C and
- for regeneration, O₂ pretreatment for 30-0.5 hours at 220-380°C and H₂ treatment for 2-6 hours at 150-250°C.

2. Process for the preparation of mixtures of cyclohexanone and cyclohexanol in the range from 30:70 to below 85:15 by hydrogenation of phenol with hydrogen in the gas phase at 150 to 250°C under 0.8 to 8 bar, preferably 1 to 5 bar, over supported palladium catalysts, characterized in that, for initial activation and for regeneration after use, the catalysts are subjected to an oxygen pretreatment and then a hydrogen treatment in each case under 0.8 to 8 bar, preferably 1 to 5 bar, particularly preferably 1 to 3 bar, the following time/temperature conditions being applied:
- for initial activation, O₂ pretreatment for 40-3 hours at 250-500°C and H₂ treatment for 2-6 hours at 150-250°C and
- for regeneration, O₂ pretreatment for 30-0.5 hours at 380-600°C and H₂ treatment for 2-6 hours at 150-250°C.

3. Process according to Claim 1 and according to Claim 2, characterized in that an inert gas/O₂ mixture, the O₂ content of which is initially 0.5 % by volume and increases to 30 % by volume during the O₂ treatment is employed for the O₂ treatment.

4. Process according to Claim 3, characterized in that the O₂ content rises to up to 21 % by volume.

5. Process according to Claim 4, characterized in that atmospheric air is first diluted with inert gas and, during the O₂ treatment, the inert gas content is reduced until the normal value of 21 % by volume of O₂ is reached.

6. Process according to Claim 1 and according to Claim 2, characterized in that the O₂ pretreatment and the H₂ treatment are carried out in the phenol hydrogenation reactor.

7. Process according to Claim 6, characterized in that the H₂ treatment is carried out in the context of regeneration under the hydrogenation operating conditions.

8. Process according to Claim 1, characterized in that the initial activation is carried out by H₂ treatment for 40 to 20 hours at 350 to 450°C, preferably for 35 to 20 hours at 370 to 450°C, and the regeneration is carried out by O₂ treatment for 25 to 1 hours at 230 to 370°C, preferably for 20 to 1 hours at 250 to 360°C, and subsequent H₂ treatment.

9. Process according to Claim 2, characterized in that the initial activation is carried out by O₂ treatment for 30 to 4 hours at 300 to 450°C, preferably for 24 to 5 hours at 350 to 425°C, and subsequent H₂ treatment and the regeneration is carried out by O₂ treatment for 25 to 1 hours at 390 to 580°C, preferably for 20 to 1 hours at 400 to 550°C, and subsequent H₂ treatment.

## Revendications

1. Procédé pour la préparation de mélanges de cyclohexanone et de cyclohexanol dans le domaine de 85:15 à 98:2 par hydrogénation de phénol avec de l'hydrogène en phase gazeuse à une température de 150 à 250°C et sous une pression de 0,8 à 8 bar, de préférence de 1 à 5 bar, sur des catalyseurs comportant un support et du palladium, caractérisé en ce qu'on soumet les catalyseurs pour une première activation et pour la régénération après utilisation, le cas échéant, à un prétraitement avec de l'oxygène et ensuite, à un traitement avec de l'hydrogène, sous une pression respectivement de 0,8 à 8 bar, de préférence de 1 à 5 bar, de manière particulièrement préférée de 1 à 3 bar, en utilisant les conditions de temps et de température ci-après:
- pour la première activation, pas de prétraitement avec O₂ et traitement avec H₂ pendant un laps de temps de 50 à 10 heures à une température de 300 à 500°C, et
- pour la régénération, prétraitement avec O₂ pendant un laps de temps de 30 à 0,5 heure à une température de 220 à 380°C et traitement avec H₂ pendant un laps de temps de 2 à 6 heures à une température de 150 à 250°C.

2. Procédé pour la préparation de mélanges de cyclohexanone et de cyclohexanol dans le domaine de 30:70 à <85:15 par hydrogénation de phénol avec de l'hydrogène en phase gazeuse à une température de 150 à 250°C et sous une pression de 0,8 à 8 bar, de préférence de 1 à 5 bar, sur des catalyseurs comportant un support et du palladium, caractérisé en ce qu'on soumet les catalyseurs pour une première activation et pour la régénération après utilisation, à un prétraitement avec de l'oxygène et ensuite, à un traitement avec de l'hydrogène, sous une pression respectivement de 0,8 à 8 bar, de préférence de 1 à 5 bar, de manière particulièrement préférée de 1 à 3 bar, en utilisant les conditions de temps et de température ci-après:
- pour la première activation, prétraitement avec O₂ pendant un laps de temps de 40 à 3 heures à une température de 250 à 500°C et traitement avec H₂ pendant un laps de temps de 2 à 6 heures à une température de 150 à 250°C, et
- pour la régénération, prétraitement avec O₂ pendant un laps de temps de 30 à 0,5 heure à une température de 380 à 600°C et traitement avec H₂ pendant un laps de temps de 2 à 6 heures à une température de 150 à 250°C.

3. Procédé selon les revendications 1 et 2, caractérisé en ce que, pour le traitement avec O₂, on met en oeuvre un mélange de gaz inerte-O₂ dont la teneur en O₂ s'élève au début à 0,5% en volume et, pendant le traitement avec O₂, augmente jusqu'à 30% en volume.

4. Procédé selon la revendication 3, caractérisé en ce que la fraction de 0₂ augmente jusqu'à concurrence de 21% en volume.

5. Procédé selon la revendication 4, caractérisé en ce qu'on dilue l'air atmosphérique tout d'abord avec un gaz inerte et on réduit la fraction en gaz inerte pendant le traitement avec O₂ jusqu'à ce que l'on atteigne la valeur normale de 21% en volume de O₂.

6. Procédé selon les revendications 1 et 2, caractérisé en ce qu'on effectue le prétraitement avec O₂ et le traitement avec H₂ dans un réacteur d'hydrogénation de phénol.

7. Procédé selon la revendication 6, caractérisé en ce qu'on effectue le traitement avec H₂ dans le cadre d'une régénération dans les conditions de service d'une hydrogénation.

8. Procédé selon la revendication 1, caractérisé en ce qu'on effectue la première activation par traitement avec H₂ pendant un laps de temps de 40 à 20 heures à une température de 350 à 400°C, de préférence pendant un laps de temps de 35 à 20 heures à une température de 370 à 450°C, et on effectue la régénération par traitement avec O₂ pendant un laps de temps de 25 à l heure à une température de 230 à 370°C, de préférence pendant un laps de temps de 20 à 1 heure à une température de 250 à 360°C, et par traitement ultérieur avec H₂.

9. Procédé selon la revendication 2, caractérisé en ce qu'on effectue la première activation par traitement avec O₂ pendant un laps de temps de 30 à 4 heures à une température de 300 à 450°C, de préférence pendant un laps de temps de 24 à 5 heures à une température de 350 à 425°C, et par traitement ultérieur avec H₂, et on effectue la régénération par traitement avec O₂ pendant un laps de temps de 25 à 1 heure à une température de 390 à 580°C, de préférence pendant un laps de temps de 20 à 1 heure à une température de 400 à 550°C, et par traitement ultérieur avec H₂.
